# EUROPÄISCHE PATENTSCHRIFT

(11) **EP 1 136 070 B1**
(45) Veröffentlichungstag und Bekanntmachung des Hinweises auf die Patenterteilung: **29.03.2006**
(21) Anmeldenummer: 01106041.5
(22) Anmeldetag: 12.03.2001
(51) Int. Cl.: A61K 9/48

(54) **Weichkapseln enthaltend Polymerisate von Vinylestern und Polyethern, deren Verwendung und Herstellung**
Soft capsules comprising polymerisates of vinylesters and polyethers, use and preparation thereof
Capsules molles comprenant des polymérisats d'esters vinyliques et de polyéthers, leur utilisation et fabrication

(30) Priorität: 14.03.2000 DE 10012063
(43) Veröffentlichungstag der Anmeldung: 26.09.2001
(73) Patentinhaber: BASF Aktiengesellschaft, 67056 Ludwigshafen (DE)
(72) Erfinder: Angel, Maximilian, Dr., 67105 Schifferstadt (DE); Kolter, Karl, Dr., 67117 Limburgerhof (DE); Sanner, Axel, Dr., 67227 Frankenthal (DE); Gotsche, Michael, Dr., 68167 Mannheim (DE)

(56) Entgegenhaltungen:
- DE-B- 1 081 229
- US-A- 3 984 494

## Beschreibung

Die vorliegende Erfindung betrifft Weichkapselhüllen, z.B. für pharmazeutische Anwendungen enthaltend Polymerisate hergestellt durch Polymerisation von Vinylestern in Gegenwart von Polyethern, sowie gegebenenfalls in Anwesenheit von strukturverbessernde Hilfsstoffen und/oder weiteren übliche Hüllbestandteilen, sowie deren Verwendung und Herstellung.

Weichkapseln zeichnen sich dadurch aus, dass die Herstellung der Hülle und das Befüllen in einem Schritt nahezu simultan erfolgen. In der Regel besteht die Hülle solcher Weichkapseln hauptsächlich aus Gelatine, weshalb die Kapseln auch häufig als Weichgelatinekapseln bezeichnet werden. Da Gelatine an sich ein sprödes, wenig flexibles Material ist, muß es entsprechend weichgemacht werden, das heißt es müssen Weichmacher zugesetzt werden. Solche Weichmacher sind niedermolekulare Verbindungen, in der Regel Flüssigkeiten, wie z.B. Glycerin, Propylenglykol, Polyethylenglykol 400. Darüber hinaus enthalten solche Kapseln oft noch Farbstoffe, Opakisierungsmittel und Konservierungsstoffe.

Gelatine wird zwar häufig eingesetzt, jedoch weist sie zahlreiche Nachteile auf. So ist Gelatine ein Material tierischen Ursprungs und damit nicht kosher. Außerdem bleibt immer ein geringes Restrisiko von BSE, da zu ihrer Herstellung bevorzugt Gelatine von Rindern verwendet wird. Die Gewinnung einer geeigneten Gelatine ist sehr aufwendig und erfordert eine strenge Überwachung des Prozesses. Trotzdem sind die Chargenunterschiede aufgrund des tierischen Ursprungs, der einer gewissen Variabilität unterliegt, groß. Gelatine ist mikrobiell sehr anfällig, da sie einen guten Nährboden für Mikroorganismen darstellt. Bei der Herstellung, wie auch der Verwendung von solchen Verpackungsmaterialien müssen deshalb entsprechende Maßnahmen ergriffen werden. Häufig ist der Einsatz von Konservierungsmitteln unerläßlich.

Die bei der Herstellung von Gelatinekapseln unbedingt erforderlichen Weichmacher treten häufig von der Hülle in das Füllgut über und führen dort zu Veränderungen. Die Hülle verarmt an Weichmachern und wird im Laufe der Lagerung spröde und mechanisch instabil. Darüber hinaus besitzt eine Weichgelatinekapsel einen relativ hohen Wassergehalt in der Hülle, der ebenfalls eine weichmachende Wirkung hat. Werden solche Kapseln bei reiner Luftfeuchte gelagert, so verdunstet Wasser aus der Hülle, wodurch die Kapsel ebenfalls versprödet. Gleiches passiert auch, wenn sehr hygroskopische Güter verkapselt werden. Besonders hygroskopische oder hydrolyseempfindliche Stoffe können überhaupt nicht verkapselt werden.

Die Lösungsgeschwindigkeit von Gelatine ist verhältnismäßig langsam. Für schnelle Wirkstofffreisetzungen wäre eine höhere Auflösungsgeschwindigkeit in Magen- bzw. Darmsaft wünschenswert.

Zahlreiche Stoffe führen mit Gelatine zu Interaktionen wie z.B. Aldehyde, Polyphenole, reduzierende Zucker, mehrwertige Kationen, Elektrolyte, kationische oder anionische Polymere etc., wobei häufig Vernetzung eintritt und die Kapsel nicht mehr oder nur noch ganz langsam zerfällt bzw. sich auflöst. Für ein Arzneimittel sind solche Veränderungen verheerend, da die Wirksamkeit nicht mehr gegeben ist. Auch viele Arzneistoffe führen mit Gelatine zu Interaktionen. Zum Teil bilden sich während der Lagerung Abbauprodukte von Arzneistoffen mit beispielsweise aldehydischer Struktur, die zu einer Vernetzung der Gelatine führen. Da Gelatine sowohl saure wie auch basische Gruppen aufweist, ist verständlich, daß Reaktionen mit anderen geladenen Molekülen leicht eintreten.

Gelatine kann enzymatisch gespalten werden. Verunreinigungen durch Enzyme bzw. von Bakterien abgesonderte Enzyme können die Eigenschaften von Gelatine dramatisch verändern.

Weichgelatinekapseln verkleben sehr leicht unter warmen und feuchten Bedingungen.

Die Haftung von Filmüberzügen auf Weichgelatinekapseln ist extrem schlecht. Häufig muß hierbei umständlich erst ein spezielles Subcoating aufgezogen werden.

Aufgrund dieser vielen Nachteile hat es nicht an Versuchen gefehlt, die Gelatine in Weichkapselhüllen ganz oder teilweise zu ersetzen.

Polyvinylalkohol ist beispielsweise für diesen Zweck beschrieben. Polyvinylalkohol weist jedoch eine langsame Lösungsgeschwindigkeit auf, erfordert ebenfalls zusätzliche Weichmacher, die wiederum migrieren können und die, wie oben bereits beschrieben, die Eigenschaften des Füllguts verändern können, und kann außerdem in Folge innerer Kristallisation stark verspröden. Insbesondere bei niedriger Umgebungsfeuchte nimmt die Flexibilität im Laufe der Lagerung dramatisch ab.

Im US-Patent 5,342,626 wird eine Kombination aus Gellan, Carrageenan und Mannan für die Herstellung von Weichkapseln oder Mikrokapseln beschrieben. Alle diese Komponenten sind natürlichen Ursprungs und unterliegen den natürlichen Qualitätsschwankungen. Niedermolekulare Weichmacher sind erforderlich und die Produkte verspröden bei niedriger Umgebungsfeuchte. Ähnliches gilt für die in der Anmeldung WO 99/07347 beschriebenen Weich- oder Hartkapseln aus Carrageenan.

In WO 91/19487 wird eine Kombination aus einem kationischen Polymer und einem anionischen Polymer beschrieben. Schon aus den angegebenen Daten ist ersichtlich, daß sich die Flexibilität stark mit der Umgebungsfeuchte verändert; sie nimmt mit niedrigerer Feuchte stark ab. Dies ist verständlich, da die Ladungen der Polymere Wasser stark anziehen. Der Grad zwischen zu klebrigen und zu spröden Polymermischungen wird als sehr schmal angegeben. Die Ladungen der Polymere können zu Interaktionen mit dem Füllmaterial und den Arzneistoffen führen, zumal die meisten Arzneistoffe ebenfalls geladen vorliegen.

In WO 99/40156 werden Kombinationen von Polyethylenglykolen unterschiedlicher Molekulargewichte beschrieben, die für die Herstellung von Filmen bzw. Weichkapseln geeignet sind. Polyethylenglykole mit hohem Molekulargewicht lösen sich aber nur langsam in Wasser auf und sind spröde. Durch die Kombination mit Polyethylenglykolen mit sehr niedrigem Molekulargewicht werden sie zwar etwas flexibler aber auch klebriger. Zudem können diese wiederum aufgrund ihres niedrigen Molekulargewichtes in das Füllgut migrieren.

Die Anmeldung WO 98/27151 beschreibt eine Mischung aus Celluloseether und Polysacchariden sowie "sequestering agents", wobei der Celluloseether den Hauptbestandteil darstellt (90 bis 99, 98 %) für die Herstellung von Hart- und Weichkapseln. Aufgrund der Sprödigkeit der Celluloseether ist diese Zubereitung ohne Weichmacher höchstens für Hartgelatinekapseln geeignet und bei Zusatz von Weichmachern treten wieder die o.g. Nachteile auf. Die Auflösungsgeschwindigkeit solcher Kapseln ist ebenfalls nicht zufriedenstellend.

DE-A2 2 363 853 beschreibt die Verwendung von teilverseiften Copolymerisaten von Vinylacetat auf Polyethylenglykol zur Herstellung von Hartkapseln für Medikamente. Für die Verwendung der Copolymerisate zur Herstellung von Weichkapseln finden sich in dieser Schrift keine Hinweise.

An Hartkapseln werden jedoch ganz andere Anforderungen gestellt als an Weichkapseln. Hartkapseln benötigen eine hohe Festigkeit während bei Weichkapseln die Flexibilität im Vordergrund steht. Auch die Herstellungsverfahren sind völlig unterschiedlich. Bei Hartkapseln wird zunächst nur die Hülle in 2 separaten Teilen, einem Oberteil und Unterteil, mittels eines Tauchverfahrens hergestellt, während bei Weichkapseln die Herstellung der Hülle und die Füllung nahezu simultan verlaufen.

Bei den Hartkapseln werden nach der Herstellung von Oberteil und Unterteil diese locker ineinandergeschoben, so daß der pharmazeutische Hersteller die beiden Teile maschinell wieder trennen kann, sein Pulver einfüllen und die Kapsel verschließen kann. Bei näherer Betrachtung dieser Verarbeitung ist klar, daß die beiden Kapselteile mechanisch sehr stabil sein müssen, zumal die Füllmaschinen sehr schnell laufen und Formveränderungen den ganzen Prozeß lahmlegen würden.

Bei Weichkapseln muß die Hülle erstens absolut dicht sein, damit das Füllgut, das in der Regel flüssig ist, nicht austreten kann, und zweitens sehr flexibel sein, weil das Füllgut sonst durch Risse bzw. Mikrorisse austreten würde. Bei der Herstellung ist eine besonders hohe Flexibilität erforderlich, weil der Polymerfilm in Hohlbohrungen eingesaugt wird und damit stark verformt und gedehnt wird. Die Herstellung von Weichkapseln ist ein technologisch enorm anspruchsvoller Prozeß, von daher müssen die Polymereigenschaften und die Maschinen exakt angepaßt und eingestellt werden.

Die völlig unterschiedlichen Verfahren zur Herstellung von Hart- und Weichgelatinekapseln sind beschrieben in W. Fahrig und U. Hofer, Die Kapsel, Wissenschaftliche Verlagsgesellschaft mbH Stuttgart, 1983, S. 58-82.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Die Anmeldung WO 97/35537 beschreibt ein spezielles Verfahren zur Herstellung von Weichkapseln unter Verwendung von verschiedenen Materialien, hauptsächlich Polyvinylalkohol. Vor der Verkapselung wird ein Lösungsmittel auf den Film aufgebracht, um ihn anzulösen, damit die Verklebung besser erfolgen kann. Dies ist allerdings nur bei entsprechend schwer zu verarbeitenden Filmen erforderlich.

Dieser Erfindung lag daher die Aufgabe zugrunde, ein Material für Weichkapselhüllen zu entwickeln, das der Gelatine und vielen bisher bekannten Ersatzmaterialien überlegen ist und insbesondere auch ohne zusätzliche Weichmacher zu verarbeiten ist.

Die Aufgabe wurde erfindungsgemäß gelöst durch Weichkapselhüllen, enthaltend
a) Polymerisate, hergestellt durch Polymerisation von Vinylestern in Gegenwart von Polyether
a) gegebenenfalls strukturverbessernde Hilfsstoffe und
b) gegebenenfalls weitere übliche Bestandteile.

Die Polymerisate (a), sind erhältlich durch radikalische Polymerisation von
a) mindestens einem Vinylester in Gegenwart von
b) polyetherhaltigen Verbindungen
und gegebenenfalls einem oder mehreren copolymerisierbaren Monomeren c) und anschließender zumindest teilweiser Verseifung der Esterfunktionen der ursprünglichen Monomere a). Bevorzugt werden die erfindungsgemäßen Weichkapseln verwendet zur Herstellung von pharmazeutischen Darreichungsformen.

Bei der Herstellung der erfindungsgemäß verwendeten Polymerisate kann es während der Polymerisation zu einer Pfropfung auf die polyetherhaltigen Verbindungen (b) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften polyetherhaltigen Verbindungen und Homo- oder Copolymerisaten der Monomeren a) und c) zu verstehen.

Als polyetherhaltige Verbindungen (b) können sowohl Polyalkylenoxide auf Basis von Ethylenoxid, Propylenoxid, Butylenoxid und weiteren Alkylenoxiden als auch Polyglycerin verwendet werden.

Je nach Art der Monomerbausteine enthalten die Polymere folgende Struktureinheiten.
-(CH₂)₂-O-, -(CH₂)₃-O-, -(CH₂)₄-O-, -CH₂-CH(R⁶)-O-, -CH₂-CHOR⁷-CH₂-O-
mit
- R⁶: C₁-C₂₄-Alkyl;
- R⁷: Wasserstoff, C₁-C₂₄-Alkyl, R⁶-C(=O)-, R⁶-NH-C(=O)-.

Dabei kann es sich bei den Struktureinheiten sowohl um Homopolymere als auch um statistische Copolymere und Blockcopolymere handeln.

Bevorzugt werden als Polyether (b) Polymerisate der allgemeinen Formel I verwendet, 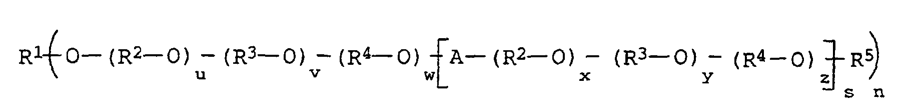 in der die Variablen unabhängig voneinander folgende Bedeutung haben:
- R¹: Wasserstoff, C₁-C₂₄-Alkyl, R⁶-C(=O)-, R⁶-NH-C(=O)-, Polyalkoholrest;
- R⁵: Wasserstoff, C₁-C₂₄-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₂₄-Alkyl;
- R⁷: Wasserstoff, C₁-C₂₄-Alkyl, R⁶-C(=O)-, R⁶-NH-C(=O)-;
- A: -C(=O)-O, -C(=O)-B-C(=O)-O, -C(=O)-NH-B-NH-C(=O)-O;
- B: -(CH₂)ₜ-, Arylen, ggf. substituiert;
- n: 1 bis 1000;
- s: 0 bis 1000;
- t: 1 bis 12;
- u: 1 bis 5000;
- v: 0 bis 5000;
- w: 0 bis 5000:
- x: 0 bis 5000;
- y: 0 bis 5000;
- z: 0 bis 5000.

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden.

Als Alkylreste für R¹ und R⁵ bis R⁷ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich kleiner 1000000 (nach Zahlenmittel), bevorzugt im Bereich von 300 bis 100000, besonders bevorzugt im Bereich von 500 bis 50000, ganz besonders bevorzugt im Bereich von 800 bis 40000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen b) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Besonders bevorzugt werden als Polyether (b) Polymerisate der allgemeinen Formel I mit einem mittleren Molekulargewicht von 300 bis 100.000 (nach dem Zahlenmittel), in der die Variablen unabhängig voneinander folgende Bedeutung haben:
- R¹: Wasserstoff, C₁-C₁₂-Alkyl, R⁶-C(=O)-, R⁶-NH-C(=O)-, Polyälkoholrest;
- R⁵: Wasserstoff, C₁-C₁₂-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₁₂-Alkyl;
- R⁷: Wasserstoff, C₁-C₁₂-Alkyl, R⁶-C(=O)-, R⁶-NH-C(=O)-;
- n: 1 bis 8;
- s: 0;
- u: 2 bis 2000;
- v: 0 bis 2000;
- w: 0 bis 2000.

Ganz besonders bevorzugt werden als Polyether b) Polymerisate der allgemeinen Formel I mit einem mittleren Molekulargewicht von 500 bis 50000 (nach dem Zahlenmittel), in der die Variablen unabhängig voneinander folgende Bedeutung haben:
- R¹: Wasserstoff, C₁-C₆-Alkyl, R⁶-C(=O), R⁶-NH-C(=O)-;
- R⁵: Wasserstoff, C₁-C₆-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₆-Alkyl;
- R⁷: Wasserstoff, C₁-C₆-Alkyl, R⁶-C(=O)-, R⁶-NH-C-=O)-;
- n: 1;
- s: 0;
- u: 5 bis 1000;
- v: 0 bis 1000;
- w: 0 bis 1000.

Des weiteren können als Polyether (b) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid(meth)acrylamide, Polyalkylenoxidallyamide oder Polyalkylenoxidvinylamide verwendet werden. Selbstverständlich können auch Copolymerisate solcher Monomere mit anderen ethylenisch ungesättigten Monomeren eingesetzt werden.

Für die Polymerisation in Gegenwart der Polyether b) seien als Komponente a) folgende radikalisch polymerisierbare Monomere genannt:

Vinylester von aliphatischen, gesättigten oder ungesättigten C₁-C₂₄-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten C₁-C₁₂-Carbonsäuren, insbesondere der C₁-C₆-Carbonsäuren, verwendet. Ganz besonders bevorzugt ist Vinylacetat.

Selbstverständlich können auch Mischungen der jeweiligen Monomeren aus der Gruppe a) copolymerisiert werden.

Die Vinylester (a) können daneben auch in Mischung mit einem oder mehreren, ethylenisch ungesättigten copolymerisierbaren Comonomeren (c) eingesetzt werden, wobei der Anteil dieser zusätzlichen Monomere auf maximal 50 Gew.-% beschränkt sein sollte. Bevorzugt sind Anteile 0 und 20 Gew.-%. Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Die bevorzugten zusätzlich eingesetzten ethylenisch ungesättigten Comonomere (c) können durch die folgende allgemeine Formel beschrieben werden:

X-C(O)CR¹⁵=CHR¹⁴

wobei
X ausgewählt ist aus der Gruppe der Reste -OH, -OM, -OR¹⁶, NH₂, -NHR¹⁶, N(R¹⁶)₂ ;
M ist ein Kation ausgewählt aus der Gruppe bestehend aus: Na⁺, K⁺, Mg⁺⁺, Ca⁺⁺, Zn⁺⁺, NH₄⁺, Alkylammonium, Dialkylammonium, Trialkylammonium und Tetraalkylammonium;
die Reste R¹⁶ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

R¹⁵ und R¹⁴ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (c) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkly)Polypropylenglykolen oder ethoxylierten Fettalkoholen, beispielsweise C₁₂-C₂₄-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (III) 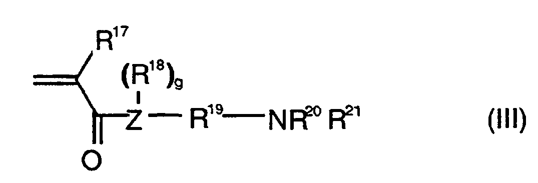 mit
- R¹⁷ =: H, Alkyl mit 1 bis 8 C-Atomen,
- R¹⁸ =: H, Methyl,
- R¹⁹ =: Alkylen mit 1 bis 24 C-Atomen, optional substituiert durch Alkyl,
- R²⁰, R²¹ =: C₁-C₄₀ Alkylrest,
- Z =: Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Comonomere der Formel III sind N,N-Dimethylaminomethyl(meth)acrylat, N,N-Diethylaminomethyl(meth)acrylat, N,N-Dimethylaminoethyl(meth)acrylat, N,N-Diethylaminoethyl(meth)-acrylat, N-[3-(dimethylamino)propyl]methacrylamid und N-[3-(dimethylamino)propyl]acrylamid.

Ebenfalls verwendbare Comonomere (c) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (c) sind Allylester von C₁-C₄₀ linearen, C₃-C₄₀ verzweigtkettigen oder C₃-C₄₀ carbocyclische Carbonsäuren, Vinyl- oder Allylhalogenide, bevorzugt Vinylchlorid und Allylchlorid, Vinylether, bevorzugt Methyl-, Ethyl-, Butyl- oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel IV geeignet, worin R²² bis R²⁴ unabhängig voneinander für Wasserstoff, C₁-C₄-Alkyl oder Phenyl steht:

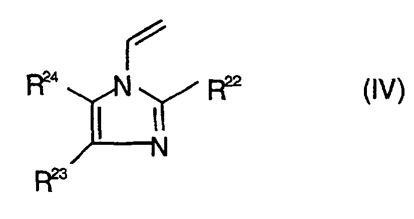

Weitere geeignete Comonomere (c) sind Diallylamine der allgemeinen Formel (V) 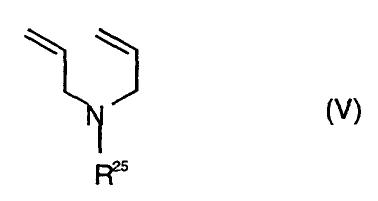 mit R²⁵ = C₁- bis C₂₄-Alkyl

Weitere geeignete Comonomere (c) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Comonomere (c) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;
Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl (meth) acrylat, N,N-Diethylaminomethyl(meth)-acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl(meth)-acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)-butyl]methacrylamid, N-[8-(dimethylamino)octyl]methacrylamid, N-[12-(dimethylamino)dodecyl]methacrylamid, N-[3-(diethylamino)-propyl]methacrylamid, N-[3-(diethylamino)propyl]acrylamid;
Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)-propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (VI) eingesetzt werden (R²⁶ = C₁- bis C₄₀-Alkyl).

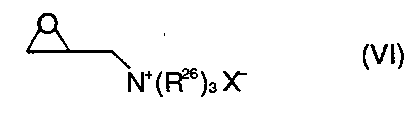

Beispiele hierfür sind zum Beispiel:
(Meth)acryloyloxyhydroxypropyltrimethylammoniumchlorid und
(Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (c) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.

Bevorzugt werden silikonfreie Regler eingesetzt.

Als zusätzliche Monomere c) können auch vernetzende Monomere eingesetzt werden. Der Begriff vernetzend bedeutet, daß die Monomere mindestens zwei nicht konjugierte, ethlylenisch ungesättigte Doppelbindungen besitzen. Geeignete Verbindungen sind beispielsweise Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z.B. Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0 bis 5 Gew.-%, ganz besonders bevorzugt 0 bis 2 Gew.-%.

Die erfindungsgemäßen Comonomere (c) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidonopropan)dihydrochlorid oder 2-2'-Azo-bis-(2-methylbutyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Bevorzugt werden organische Peroxide eingesetzt.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind.

Bei der Polymerisation in Substanz kann man so vorgehen, daß man die polyetherhaltige Verbindung b) in mindestens einem Monomer der Gruppe a) und gegebenenfalls eines oder mehreren Comonomeren der Gruppe c) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der polyetherhaltigen Verbindung b), mindestens einem Monomeren der Gruppe a), gegebenenfalls eines oder mehreren Comonomeren der Gruppe c) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die polyetherhaltigen Verbindungen der Gruppe b) in einem Reaktor vorlegt, auf die Polymerisationstemperatur erwärmt und mindestens ein Monomer der Gruppe a), gegebenenfalls eines oder mehreren Comonomeren der Gruppe c) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Polymerisation von
a) 10 bis 98 Gew.-% mindestens eines Vinylesters von C₁-C₂₄-Carbonsäuren in Gegenwart von
b) 2 bis 90 Gew.-% mindestens einer polyetherhaltigen Verbindung und
c) 0 bis 50 Gew.-% eines oder mehreren weiteren copolymerisierbaren Monomeren

Besonders bevorzugt sind Polymere, die erhältlich sind durch radikalische Polymerisation von
a) 50 bis 97 Gew.-% mindestens eines Vinylesters von C₁-C₂₄-Carbonsäuren in Gegenwart von
b) 3 bis 50 Gew.-% mindestens einer polyetherhaltigen Verbindung und
c) 0 bis 20 Gew.-% eines oder mehreren weiteren copolymerisierbaren Monomeren

Ganz besonders bevorzugt sind Polymere, die erhältlich sind durch radikalische Polymerisation von
a) 65 bis 97 Gew.% mindestens eines Vinylesters von C₁-C₂₄-Carbonsäuren in Gegenwart von
b) 3 bis 35 Gew.% mindestens einer polyetherhaltigen Verbindung und
c) 0 bis 20 Gew.% eines oder mehreren weiteren copolymerisierbaren Monomeren

Zur Herstellung der erfindungsgemäß verwendeten Polymeren werden die Estergruppen der ursprünglichen Monomere a) und gegebenenfalls weiterer Monomere nach der Polymerisation durch Hydrolyse, Alkoholyse oder Aminolyse gespalten. Im Nachfolgenden wird dieser Verfahrensschritt allgemein als Verseifung bezeichnet. Die Verseifung erfolgt in an sich bekannter Weise durch Zugabe einer Base, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base, von der Verseifungstemperatur, der Verseifungszeit und dem Wassergehalt der Lösung.

Der Verseifungsgrad der Polyvinylestergruppen liegt im Bereich von 1 bis 100 %, bevorzugt im Bereich von 40 bis 100 %, besonders bevorzugt im Bereich von 65 bis 100 %, ganz besonders bevorzugt im Bereich von 80 bis 100 %.

Die so hergestellten Polymerisate können durch Umsetzung von im Polymer vorhandenen Hydroxyl- und/oder Aminofunktionen mit Epoxiden der Formel VI nachträglich kationisiert werden (R²⁶ = C₁ bis C₄₀ Alkyl).

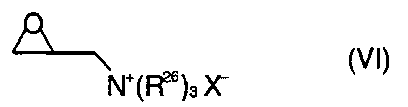

Dabei können bevorzugt die Hydroxylgruppen der Polyvinylalkohol-Einheiten und Vinylamin-Einheiten, entstanden durch Hydrolyse von Vinylformamid, mit den Epoxiden umgesetzt werden.

Die Epoxide der Formel VI können auch in situ durch Umsetzung der entsprechenden Chlorhydrine mit Basen, beispielsweise Natriumhydroxid, erzeugt werden.

Bevorzugt wird 2,3-Epoxypropyl-trimethylammoniumchlorid bzw. 3-Chlor-2-hydroxypropyl-trimethylammoniumchlorid eingesetzt.

Die K-Werte der Polymerisate sollen im Bereich von 10 bis 300, bevorzugt 25 bis 250, besonders bevorzugt 25 bis 200, ganz besonders bevorzugt im Bereich von 30 und 150, liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25°C und Polymerkonzentrationen, die je nach K-Wert-Bereich zwischen 0,1 Gew.-% und 5 Gew.-% liegen.

Nach der Verseifung können die Polymerlösungen zur Entfernung von Lösungsmitteln wasserdampfdestilliert werden. Nach der Wasserdampfdestillation erhält man je nach Verseifungsgrad, Art der Polyether b), der Vinylester a) und der eventuell eingesetzten Monomere c) wäßrige Lösungen oder Dispersionen.

Die erhaltenen Polymerisate können auch nachträglich vernetzt werden, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgrade wasserquellbare bzw. unlösliche Produkte.

Beispielsweise können die erfindungsgemäßen Polymerisate mit Dialdehyden und Diketonen, z.B. Glyoxal, Glutaraldehyd, Succindialdehyd oder Terephthalaldehyd, umgesetzt werden. Desweiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B. Epichlorhydrin, Glycidylmethacrylat, Ethylenglykoldiglycidylether, 1,4-Butandioldiglycidylether oder 1,4-Bis(glycidyloxy)benzol. Ferner eigenen sich Diisocyanate, beispielsweise Hexamethylendiisocyanat, Isophorondiisocyanat, Methylendiphenyldiisocyanat, Toluylendiisocyanat oder Divinylsulfon.

Weiterhin eignen sich anorganische Verbindungen wie Borsäure oder Borsäuresalze, beispielsweise Natriummetaborat, Borax (Dinatriumtetraborat), sowie Salze mehrwertiger Kationen, z.B. Kupfer-(II)salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.
Die Borsäure bzw. Borsäuresalze können den Polymerlösungen direkt nach der Herstellung zugefügt werden. Es ist aber auch möglich, die Borsäure bzw. Borsäuresalze nachträglich den erfindungsgemäßen Polymerisaten zuzusetzen, bzw. während des Herstellungsprozesses der Weichkapseln.

Der Anteil Borsäure bzw. Borsäuresalze bezogen auf die erfindungsgemäßen Polymere beträgt 0 bis 15 Gew-%, bevorzugt 0 bis 10 Gew.-%, besonders bevorzugt 0 bis 5 Gew.-%.

Die Lösungen und Dispersionen der erfindungsgemäßen Polymerisate können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Anstelle der wasserdampfdestillierten Polymerlösungen können auch die alkoholischen Polymerlösungen direkt in Pulverform überführt werden.

Die erfindungsgemäßen wasserlöslichen oder wasserdispergierbaren Polymerisate eignen sich hervorragend zur Herstellung von Weichkapselhüllen, insbesondere für pharmazeutische Darreichungsformen.

Die erfindungsgemäßen Polymere, hergestellt durch radikalische Polymerisation von Vinylestern und gegebenenfalls eines oder mehreren polymerisierbaren Monomeren in Gegenwart polyetherhaltiger Verbindungen und anschließender zumindest teilweiser Verseifung der Esterfunktionen der ursprünglichen Vinylester, eignen sich zur Herstellung von Weichkapseln.

Die Polymerisate lassen sich mit den o.g. Verfahren mit hoher Reproduzierbarkeit herstellen. Zu ihrer Herstellung werden keine Materialien tierischen Ursprunges verwendet und da auch keine pflanzlichen Materialien eingesetzt werden, stellt sich das Problem von Produkten gentechnologischen Ursprungs nicht.

Mikrobiologisch sind die Polymerisate nicht besonders anfällig, weil sie keinen guten Nährboden für Gelatine darstellen. Weder durch Enzyme noch durch Hydrolyse werden die Polymerketten abgebaut. Daher ist auch die Herstellung von Lösungen für die Filmherstellung und Verkapselung unproblematisch.

Die besondere Eignung der beschriebenen Polymere für die Herstellung von Weichkapselhüllen liegt in ihrer Flexibilität und Weichheit. Aufgrund dieser enormen Flexibilität ist der Einsatz von niedermolekularen Weichmachern in der Regel überflüssig. Daher findet auch keine Veränderung der Hülle und des Kapselinhaltes aufgrund von Migration statt.

Typische verpackte Materialien sind bevorzugt pharmazeutische Erzeugnisse, wie feste und flüssige Wirkstoffe, aber auch Vitamine, Carotinoide, Mineralstoffe, Spurenelemente, Nahrungsergänzungsstoffe, Gewürze sowie Süßstoffe. Weiterhin können die Kapseln für kosmetische Wirkstoffe ("personal care"), wie beispielsweise Haar- und Hautformulierungen, für Öle, Duftstoffe, Badezusätze oder Proteine verwendet werden. Weitere Anwendungen im Bereich "personal care" sowie weitere Anwendungen für wasserlösliche Verpackungen sind in der WO 99/40156 genannt.

Weitere verpackte Materialien können sein, z.B. Reinigungsmittel, wie Seifen, Waschmittel, Farb- und Bleichmittel, Agrarchemikalien wie Düngemittel (-kombinationen), Pflanzenschutzmittel wie Herbizide, Fungizide oder Pestizide und Saatgut.

Generell lassen sich mit den erfindungsgemäßen Polymerisaten Inhaltsstoffe verpacken, die geschützt werden sollen, bevor sie in eine wässrige Umbebung gebracht werden.

**Tabelle 1 Flexibilität von Polymeren (23°C / 54 % r. F.)**

| Zusammensetzung | Reißdehnung |
|---|---|
| PEG 6 000 / Polyvinylacetat (15 : 85), verseift | 121 % |
| PEG 6 000 / Polyvinylacetat 10 : 90 | 140 % |
| PEG 6 000 / Polyvinylacetat 5 : 95 | 209 % |

Die Bestimmung erfolgte an Filmstücken auf einem Zugprüfgerät (Texture Analyzer TA.XT 2; Winopal Forschungsbedarf GmbH, 30161 Hannover) gemäß DIN 53504.

Überraschenderweise verändert sich die Flexibilität bei Veränderung der Umgebungsfeuchte nur gering. Das heißt, daß bei Lagerung in trockener Umgebung die Weichkapseln nicht verspröden und ihre mechanische Stabilität beibehalten.

**Tabelle 2 Flexibilität von Polymeren bei unterschiedlichen Umgebungsfeuchten (23°C)**

| | Reißdehnungen [%] bei verschiedenen rel. Luftfeuchten | | | | |
|---|---|---|---|---|---|
| | 11 % | 33 % | 54 % | 65 % | 75 % |
| PEG 6 000 / Polyvinylacetat (15 : 85), verseift | 107 | 113 | 115 | 108 | 103 |
| Polyvinylalkohol (Mowiol 4/88) | 4 | 40 | 106 | - | - |
| Gelatine 200 Bloom | 0 | - | 0 | - | - |
| Gelatine 200 Bloom + 5 % Glycerin | 3 | - | 5 | - | - |
| Gelatine 200 Bloom + 35 % Glycerin | 31 | - | 157 | - | - |

Auch wenn Stoffe mit hoher Hygroskopizität verkapselt werden, bleibt die Elastizität erhalten. Die Polymere sind daher besonders geeignet für die Verkapselung von wasserempfindlichen Stoffen.

Die Auflösungsgeschwindigkeit der erfindungsgemäßen Polymere und daraus hergestellter Weichkapselhüllen ist enorm hoch und übertrifft die von Gelatine und Polyvinylalkohol deutlich. Außerdem sind die Polymere kaltwasserlöslich. Gelatine und Polyvinylalkohol lösen sich erst bei höheren Temperaturen. Da viele Arzneistoffe schnell nach der Einnahme wirken sollen, ist dieses Lösungsverhalten insbesondere für diese Verwendung ein klarer Vorteil.

**Tabelle 3 Auflösungsgeschwindigkeit von Polymeren**

| Produkt | 0,08 N-HCl | Puffer pH 6,8 |
|---|---|---|
| PEG 6 000 / Polyvinylacetat (15 : 85), verseift | 58 s | 1 min 00 s |
| Gelatine 200 Bloom + 35 % Glycerin | 1 min 20 | 1 min 31 |
| (Hydroxypropylmethylcellulose) Pharmacoat 606 | 6 min 21 s | 6 min 31 s |
| Polyvinylalkohol (Mowiol 8/88) | 3 min 10 s | 3 min 18 s |

Die Bestimmung der Auflösungsgeschwindigkeit erfolgte in einer Freisetzungsapparatur (Pharmatest PTS) nach USP 23 in einen Diarahmen mit einer lichten Weite von 3,5 x 2,5 cm eingespannten, 100 µm dicken Filmes bei 50 rpm und 37°C. Angegeben ist die Zeit, in der sich das Filmstück aufgelöst hat.

Im Gegensatz zu Gelatine können in die erfindungsgemäßen Hüllen auch Stoffe verkapselt werden, die zu Interaktionen neigen, wie z.B. Aldehyde oder mehrwertige Kationen. Eine Vernetzung und Verlängerung der Auflösungsgeschwindigkeit ist nicht zu erkennen.

Weichkapselhüllen der erfindungsgemäßen Zusammensetzung lassen sich hervorragend unter Verwendung von wäßrigen Polymerlösungen oder Polymersuspensionen coaten. So kann durch Aufsprühen von Kollicoat MAE 30 DP (Methacrylsäure-Copolymer Typ C der USP) in einem Horizontaltrommelcoater ein stark auf der Oberfläche haftender magensaftresistenter Überzug aufgebracht werden, der zudem lagerungsstabil ist.

Zur Erzielung einer Magensaftresistenz können in der Hülle außerdem 20 bis 80 %, vorzugsweise 30 bis 70 % eines magensaftresistenten Polymers enthalten sein.

Den Polymerisaten können strukturverbessernde Hilfsstoffe zugesetzt werden, um die mechanischen Eigenschaften wie Flexibilität und Festigkeit zu modifizieren. Diese strukturverbessernden Hilfsstoffe lassen sich in 2 große Gruppen einteilen.
A) Polymere mit einem Molekulargewicht größer 50000, vorzugsweise größer 100000
B) Stoffe die zu einer Vernetzung der Polymerketten entweder der Polymeren oder der unter A) genannten Stoffe führen, vorzugsweise Aldehyde, Borsäure und ihre Salze,
sowie gegebenenfalls Stoffe, die zu einer Vernetzung der Polymerketten der strukturverbessernden Hilfsstoffen führen, vorzugsweise Erdalkaliionen, Amine, Tannine sowie Aldehyde und Borate.

Als Polymere mit hohem Molekulargewicht können Stoffe aus folgenden Stoffklassen eingesetzt werden:

Polyaminosäuren, wie Gelatine, Zein, Sojaprotein sowie Derivate davon,
Polysaccharide wie Stärke, abgebaute Stärke, Maltodextrine, Carboxymethylstärke, Cellulose, Hydroxypropylmethylcellulose, Hydroxypropylcellulose, Hydroxyethylcellulose, Methylcellulose, Carboxymethylcellulose, Ethylcellulose, Celluloseacetat, Celluloseacetatphthalat, Hydroxypropylcelluloseacetatphthalat, Hydroxypropylcelluloseacetatsuccinat, Hemicellulose, Galactomannane, Pectine, Alginate, Carrageenane, Xanthan, Gellan, Dextran, Curdlan, Pullulan, Chitin, sowie Derivate davon, synthetische Polymere wie Polyacrylsäure, Polymethacrylsäure, Copolymerisate aus Acrylsäure- und Methacrylsäureestern, Polyvinylalkohole, Polyvinylacetat, Polyethylenglykole, Polyoxyethylen-Polyoxypropylen-Blockpolymere, Polyvinylpyrrolidone sowie Derivate davon.

Diese Polymere mit hohem Molekulargewicht bilden ein Netzwerk mit den Polymeren und erhöhen so die Festigkeit der Weichkapselhüllen. Die Flexibilität leidet, sofern keine sehr hohen Konzentrationen verwendet werden in aller Regel nicht. Überraschenderweise sind hierfür nicht nur wasserlösliche, sondern auch wasserunlösliche Polymere wie Copolymere aus Acrylsäure- und Methacrylsäureestern geeignet. Bleibt die Konzentration dieser wasserunlöslichen Polymere unter 50 %, zerfallen die Kapseln immer noch.

In ähnlicher Weise wirken Stoffe, die zu einer Vernetzung entweder der Polymerketten der Polymere oder der zugesetzten hochmolekularen Polymere führen.

Neben den genannten Komponenten können die erfindungsgemäßen Weichkapselhüllen noch weitere übliche Bestandteile enthalten. Dazu zählen Füllstoffe, Formtrennmittel, Rieselhilfsmittel, Stabilisatoren sowie wasserlösliche oder wasserunlösliche Farbstoffe, Aromen und Süßstoffe.

Farbstoffe sind z.B. Eisenoxide, Titandioxid, die in einer Menge von etwa 0.001 bis 10, vorzugsweise von 0.5 bis 3 Gew.% zugesetzt werden, Triphenylmethanfarbstoffe, Azofarbstoffe, Chinolinfarbstoffe, Indigofarbstoffe, Carotinoide, um die Kapseln einzufärben, Opakisierungsmittel wie Titandiodid oder Talkum, um die Lichtundurchlässigkeit zu erhöhen und um Farbstoffe einzusparen.

Aromen und Süßstoffe sind insbesondere dann von Bedeutung, wenn ein schlechter Geruch oder Geschmack überdeckt werden soll und die Kapsel zerbissen wird.

Konservierungsmittel sind in aller Regel nicht erforderlich.

Füllstoffe sind z.B. anorganische Füllstoffe wie Oxide von Magnesium, Aluminium, Silicium, Titan oder Calciumcarbonat. Der bevorzugte Konzentrationsbereich für die Füllstoffe ist etwa 1 bis 50 Gew.-%, besonders bevorzugt 2 bis 30 Gew.-% bezogen auf das Gesamtgewicht sämtlicher Komponenten.

Schmiermittel sind Stearate von Aluminium, Calcium, Magnesium und Zinn, sowie Magnesiumsilikat, Silikone und ähnliche. Der bevorzugte Konzentrationsbereich ist etwa 0.1 bis 5 Gew.-%, besonders bevorzugt etwa 0.1 bis 3 Gew.-% bezogen auf das Gesamtgewicht sämtlicher Komponenten.

Rieselhilfsmittel sind z.B. feinteilige bzw. feinstteilige Kieselsäuren, ggf. modifiziert. Der bevorzugte Konzentrationsbereich ist 0.05 bis 3 Gew.-%, besonders bevorzugt 0.1 bis 1 Gew.-% bezogen auf das Gesamtgewicht sämtlicher Komponenten.

Ein Sonderfall stellt die Einarbeitung von Wirkstoffen in die Hülle dar. Dies kann vorteilhaft sein, um inkompatible Wirkstoffe voneinander zu trennen. Der Wirkstoff mit der geringsten Dosierung sollte dann in die Hülle eingearbeitet werden.

Die Hülle der erfindungsgemäßen Verpackungsmaterialien besteht aus 10 bis 100 %, vorzugsweise 20 bis 98 % Polymerisaten, gegebenenfalls 0 bis 80 %, vorzugsweise 1 bis 50 % strukturverbessernden Hilfsstoffen und gegebenenfalls 0 bis 30 %, vorzugsweise 0,1 bis 30 % weiteren üblichen Bestandteilen.

Die Herstellung der Verpackungsmaterialien erfolgt nach üblichen Verfahren, z.B. dem "rotary-die-Verfahren", dem Accogel-Verfahren, dem Norton-Verfahren, dein Tropf- oder Blasverfahren oder nach dem Colton-Upjohn-Verfahren. Diese Verfahren sind beschrieben in W. Fahrig und U. Hofer, Die Kapsel, Wissenschaftliche Verlagsgesellschaft mbH Stuttgart, 1983.

Herstellvorschriften für die Polymerisate:

In einem Polymerisationsgefäß wird die polyetherhaltige Verbindung vorgelegt und unter Rühren und leichtem Stickstoffstrom auf 80°C erhitzt. Unter Rühren werden Vinylacetat und das weitere Monomere in 3 h zudosiert. Gleichzeitig wird eine Lösung von 1,4 g tert.-Butylperpivalat in 30 g Methanol ebenfalls in 3 h zugegeben. Danach wird noch 2 h bei 80°C gerührt. Nach dem Abkühlen wird das Polymerisat in 450 ml Methanol gelöst. Zur Verseifung gibt man bei 30°C, 50 ml einer 10%igen methanolischen Natriumhydroxidlösung zu. Nach ca. 40 min. wird die Reaktion durch Zugabe von 750 ml 1%iger Essigsäure abgebrochen. Das Methanol wird durch Destillation entfernt.

Die K-Werte wurden 1%ig in N-Methylpyrrolidon bestimmt.

**Tabelle 4**

| Beispiel | pfropfgrundlage | Vinylester | Comonomer | K-Wert | Verseifungsgrad [%] |
|---|---|---|---|---|---|
| 1 | PEG 1500¹ 72 g | Vinylacetat, 410 g | - | 47 | > 95 |
| 2 | PEG 4000 72 g | Vinylacetat, 410 g | - | 51 | > 95 |
| 3 | PEG 6000, 72 g | Vinylacetat, 410 g | - | 54 | > 95 |
| 4 | PEG 6000, 137 g | Vinylacetat, 410 g | | 49 | > 95 |
| 5 | PEG 6000, 22 g | Vinylacetat 410 g | - | 73 | > 95 |
| 6 | PEG 6000, 410 g | Vinylacetat 410 g | | 42 | > 95 |
| 7 | PEG 9000, 137 g | Vinylacetat, 410 g | - | 58 | > 95 |
| 8 | Polyglycerin 2200, 72 g | Vinylacetat, 410 g | - | 66 | > 95 |
| 9 | PEG-PPG-Blockcopolymer 8000², 72g | Vmylacetat, 410g | - | 45 | > 95 |
| 10 | Methylpolyethylenglykol 2000³ 72 g | Vinylacetat, 410 g | - | 47 | > 95 |
| 11 | Alkylpolyethylenglykol 3500⁴ 72 g | Vinylacetat, 410 g | - | 48 | > 95 |
| 12 | PPG 4000⁵ 72 | Vinylacetat 410 g | | 50 | > 95 |
| 13 | PEG 20000 72 g | Vinylacetat, 410 g | - | 69 | > 95 |
| 14 | PEG 20000 103 g | Vinylacetat, 410 g | - | 64 | > 95 |
| 15 | PEG 20000 137 g | Vinylacetat, 410 g | - | 59 | > 95 |
| 16 | PEG 20000 615 g | Vinylacetat, 410 g | - | 55 | 86 |
| 17 | PEG 35000 72 g | Vinylacetat, 410 g | - | 77 | > 95 |
| 18 | PEG 35000 137g | Vinylacetat, 410 g | - | 80 | > 95 |
| 19 | PEG 35000 205 g | Vinylacetat, 410 g | - | 65 | 97 |
| 20 | Dimethicone copolyol⁶, 202 g | Vinylacetat, 410 g | - | 58 | > 95 |
| 21 | Poly(Natriummethacrylat-co-methylpolyethylenglykolmethacrylat)⁷ 103 g, | Vinylacetat, 410 g | | 43 | > 95 |
| 22 | ethoxyliertes Polyethylenimin⁸ | Vinylacetat, | | 52 | > 95 |
| 23 | PEG 6000, 72 g | Vinylacetat, 386 g | Methylmethacrylat, 24 g | 47 | > 95 |
| 24 | PEG 20000, 72 g | Vinylacetat, 328 g | N-Vinylpyrrolidon, 82 g | 61 | > 95 |
| 25 | PEG 20000, 72 g | Vinylacetat, 362g | 3-Methyl-1-vinylimidazoliummethylsulfat, 48 g | 53 | 95 |
| 26 | PEG 6000, 72 g | Vinylacetat, 367 g | N-Vinylformamid, 41 g | 57 | > 95 |
| 27 | PEG 6000, 72 g | Vinylacetat, 326 g | N-Vinylformamid, 82 g | 67 | > 95 |
| 28 | PEG 35000, 270 g | Vinylacetat, 410 g | | 59 | 96 |
| 29 | PEG 35000, 270 g | Vinylacetat, 410 g | Pentaerythrittriallylether, 1,6 g | 71 | 95 |
| 30 | PEG 35000, 270 g 270 g | Vinylacetat, 410 g | Pentaerythrittriallylether, 0,8 g | 65 | 94 |
| 31 | PEG 35000, PEG 35000, 270g | Vinylacetat, 410 g | NN'-Divinylethylenharnstoff 0,7 g | 73 | 95 |
| 32 | PEG 12000, 270 g | Vinylacetat, 410 g | Pentaerythrittriallylether, 1,6 g | 50 | 94 |

1 PEG x: Polyethylenglykol mit mittlerem Molekulargewicht x
2 Lutrol F 68 der Fa. BASF Aktiengesellschaft (PPG: Polypropylenglykol)
3 Pluriol A 2000 E der Fa. BASF Aktiengesellschaft
4 Lutensol AT 80 der Fa. BASF Aktiengesellschaft (C₁₆-C₁₈-Fettalkohol + 80 EO)
5 Polypropylenglykol mit mittlerem Molekulargewicht 4000
6 Belsil DMC 6031TM der Fa. Wacker Chemie GmbH
7 Molverhältnis Natriummethacrylat/Methylpolyethylenglykolmethacrylat 4:1; Methylpolyethylenglykol mit Molmasse ca. 1000
8 hergestellt aus 12,5 % Polyethylenimin (mittleres Molekulargewicht 1400) und 87,5 % Ethylenoxid

### Beispiel 33: Umsetzung mit 3-Chlor-2-hydroxypropyl-trimethylammoniumchlorid

Zu 400 g einer 32,9%igen Lösung aus Beispiel 3 gibt man 22 g einer 60%igen wässerigen Lösung von 3-Chlor-2-hydroxypropyltrimethylammoniumchlorid sowie 3,5 g Natriumhydroxid. Man rührt 3 Stunden bei 60°C und anschließend zwei weitere Stunden bei 90°C. Man erhält eine klare Lösung.

### Beispiel 34: Umsetzung mit 3-Chlor-2-hydroxypropyl-trimethylammoniumchlorid

Zu 400 g einer 15,3%igen Lösung aus Beispiel 26 gibt man 46 g einer 60%igen wässerigen Lösung von 3-Chlor-2-hydroxypropyltrimethylammoniumchlorid sowie 6 g Natriumhydroxid. Man rührt 3 Stunden bei 60°C und anschließend zwei weitere Stunden bei 90°C. Man erhält eine klare Lösung.

### Beispiel 35: Nachträgliche Vernetzung mit Borax

Man gibt bei Raumtemperatur unter Rühren innerhalb einer halben Stunde zu einer 19,3 %igen wässerigen Lösung des Polymers aus Beispiel 28 eine 5%ige wässerige Lösung von Dinatriumtetraborat (Borax). Man beobachtet einen Viskositätsanstieg.

| Menge an zugesetzter 5%iger Borax-Lösung [g] | Brookfieldviskosität (LVF, Spindel 2, 30 UpM, 23°C) [mPas] |
|---|---|
| 0 | 110 |
| 14,9 | 128 |
| 18,0 | 216 |
| 21,0 | 534 |
| 24,0 | 2228 |
| 26,9 | 7520¹ |
| 29,8 | 29190² |
| | |

| | |
|---|---|
| ¹ Spindel 4, 30 UpM | |
| ² Spindel 4, 6 UpM | |

### Beispiel 36

1,0 kg Polymer aus Polyethylenglykol 6000 / Polyvinylacetat (15 : 85) verseift, wurden in 1,5 kg demineralisiertem Wasser gelöst und die auf 60°C erwärmte Lösung zu einem 300 µm dicken Film ausgezogen und bei 60°C getrocknet. Aus diesem Film wurden mittels des rotary die-Verfahrens Weichkapseln mit einer Füllung aus Vitamin E (2 Teile) und mittelkettigen Triglyceriden (8 Teile) hergestellt. Die Kapseln wurden anschließend in einem wirbelbett schonend bei 35°C nachgetrocknet.

Die Auflösung in künstlichem Magensaft betrug 2 min 30 s. Während der Lagerung bei 11 % r. F. behielten die Kapseln ihre Flexibilität und Zerfallseigenschaften.

### Beispiel 37

0,66 kg Polymer aus Polyethylenglykol 6000 / Polyvinylacetat (15/85) verseift, 0,04 kg Pectin und 0,16 kg Polyvinyalkohol (Mowiol 4/88) wurden unter Erwärmen in 1,58 kg demineralisiertem Wasser gelöst. Aus 16 g rotem Eisenoxid (Sicovit rot 30, BASF Aktiengesellschaft) und 33 g Tiandioxid wurde mit 115 g demineralisiertem Wasser eine Pigmentsuspension hergestellt, die nach der Homogenisierung über eine Korundscheibenmühle der Polymerlösung unter Rühren zugegeben wird. Die Suspension wurde zu einem Film mit 400 µm Dicke ausgezogen. Aus diesem Film wurden mittels des rotary die-Verfahrens Weichkapseln mit einer Füllung aus Ibuprofen (3 Teile)kettigen Triglyceriden (7 Teile) hergestellt. Die Kapseln wurden anschließend in einem Wirbelbett schonend bei 35°C nachgetrocknet.

Die Auflösungszeit der Kapseln in künstlichem Magensaft betrug 3 min 03 s. Während der Lagerung über 3 Monate bei 11 % r. F. behielten die Kapseln ihre Flexibilität und Zerfallseigenschaften.

### Beispiel 38

0,6 kg Polymer aus Polyethylenglykol 6000 / Polyvinylacetat (15/85) verseift, 0,5 kg Gelatine 200 Bloom und 10 g 10%iges Betacarotin-Trockenpulver (Lucarotin 10 % CWD) wurden unter Erwärmen in 1,4 kg demineralisiertem Wasser und 0,10 kg Glycerin gelöst. Die Lösung wurde zu einem Film mit 400 µm Dicke ausgezogen.

Aus diesem Film wurden mittels des rotary die-Verfahrens Weichgelatinekapseln mit einer Füllung aus Ibuprofen (3 Teile)kettigen Triglyceriden (7 Teile) hergestellt. Die Kapseln wurden anschließend in einem Wirbelbett schonend bei 35°C nachgetrocknet.

Die Auflösungszeit der Kapseln in künstlichem Magensaft betrug 4 min 30 s.

### Beispiel 39

0,175 kg eines Copolymerisates aus Methacrylsäure und Ethylacrylat (Kollicoat MAE 100 P) wurde in 1,625 kg Wasser dispergiert und unter Rühren durch Zugabe von 20 %iger Natronlauge auf pH 6,5 eingestellt. Anschließend wurden 0,7 kg Polymer aus Methylpolyethylenglykol 6000 / Polyvinylacetat (15/85) verseift unter Rühren aufgelöst. Die Lösung wurde zu einem Film mit 350 µm Dicke ausgezogen.

Aus diesem Film wurden mittels des rotary die-Verfahrens Weichkapseln mit einer Füllung aus Verapamil-HCl (3 Teile), Cremophor RH 40 (1 Teil) und mittelkettigen Triglyceriden (6 Teile) hergestellt. Die Kapseln wurden anschließend in einem Wirbelbett schonend bei 35°C nachgetrocknet.

Die Auflösungszeit der Kapseln in künstlichem Magensaft betrug 3 min 45 s.

### Beispiel 40

0,95 kg Polymer aus Polyethylenglykol 6000 / Polyvinylacetat (15/85) verseift, 0,1 kg Hydroxypropylmethylcellulose (Pharmacoat 606), 0,05 kg Carrageenan und 10 g 10 %iges Betacarotin-Trockenpulver (Lucarotin 10 % CWD) und 0,1 kg Polyethylenglykol 6000 wurden unter Erwärmen in 1,4 kg demineralisiertem Wasser gelöst. Die Lösung wurde zu einem Film mit 350 µm Dicke ausgezogen.

Aus diesem Film wurden mittels des Accogel-Verfahrens durch Einmulden des Filmes, Einspritzen der Füllung und Verschließen mit einem 2. Film Weichkapseln mit einer Füllung aus Theophyllin (3 Teile), Polysorbat 80 (0,5 Teile) und mittelkettigen Triglyceriden (6 Teile) hergestellt. Die Kapseln wurden anschließend in einem Wirbelbett schonend bei 35°C nachgetrocknet.

Die Auflösungszeit der Kapseln in künstlichem Magensaft betrug 2 min 55 s. Ein Verspröden war selbst bei einer Lagerung bei 11 % Umgebungsfeuchte nicht festzustellen.

### Beispiel 41

1,0 kg Polymer aus Polyethylenglykol 6000 / Polyvinylacetat (15/85) verseift wurden in 2,3 kg demineralisiertem Wasser gelöst und mit 10 g Natriumtetraborat versetzt. Die Lösung wurde zu einem Film mit 400 µm Dicke ausgezogen. Aus diesem Film wurden mittels des Accogel-Verfahrens durch Einmulden des Filmes, Einspritzen der Füllung und Verschließen mit einem 2. Film Weichkapseln mit einer Füllung aus Tocopherolacetat (3 Teile), Polysorbat 80 (0,5 Teile) und mittelkettigen Triglyceriden (6,5 Teile) hergestellt. Die Kapseln wurden anschließend in einem Wirbelbett schonend bei 35°C nachgetrocknet. Die Auflösungszeit der Kapseln in künstlichem Magensaft betrug 4 min 35 s. Ein Verspröden war selbst bei einer Lagerung bei 11 % Umgebungsfeuchte nicht festzustellen.

### Vergleichsbeispiel

Ohne Weichmacherzusatz konnten weder mit Gelatine noch mit Polyvinylalkohol oder Hydroxypropylmethylcellulose Weichkapseln hergestellt werden. Die Filme waren zu spröde und brüchig.

## Patentansprüche

1. Weichkapselhüllen enthaltend
(a) Polymerisate hergestellt durch Polymerisation von Vinylestern in Gegenwart von Polyethern
(b) gegebenenfalls strukturverbessernde Hilfsstoffe und
(c) gegebenenfalls weitere übliche Bestandteile.

2. Weichkapselhüllen gemäß Anspruch 1, **dadurch gekennzeichnet, daß** die Polymerisate (a) erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren copolymerisierbaren Monomeren
und anschließender zumindest teilweiser Verseifung der Esterfunktionen der ursprünglichen Monomeren a).

3. Weichkapselhüllen gemäß einem der Ansprüche 1 oder 2, **dadurch gekennzeichnet, daß** die Polymerisate (a) erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I 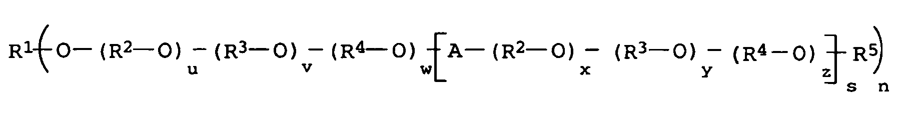 in der die Variablen unabhängig voneinander folgende Bedeutung haben:
R¹ Wasserstoff, C₁-C₂₄-Alkyl, R⁶-C(=O)-, R⁶-NH-C(=O)-, Polyalkoholrest;
R⁵ Wasserstoff, C₁-C₂₄-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-, -CH₂-CHOR⁷-CH₂- ;
R⁶ C₁-C₂₄-Alkyl;
R⁷ Wasserstoff, C₁-C₂₄-Alkyl, R⁶-C(=O)-, R⁶-NH-C(=O)-;
A -C(=O)-O, -C(=O)-B-C(=O)-O, -C(=O)-NH-B-NH-C(=O)-O;
B -(CH₂)ₜ-, Arylen, ggf. substituiert
n 1 bis 1000;
s 0 bis 1000;
t 1 bis 12;
u 1 bis 5000;
v 0 bis 5000;
w 0 bis 5000;
x 0 bis 5000;
y 0 bis 5000;
z 0 bis 5000;
und
c) gegebenenfalls eines oder mehreren weiteren copolymerisierbaren Monomeren
und anschließender zumindest teilweiser Verseifung der Esterfunktionen der ursprünglichen Monomeren a).

4. Weichkapselhüllen gemäß einem der Ansprüche 1 bis 3, **dadurch gekennzeichnet, daß** die Polymerisate (a) erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I mit einem mittleren Molekulargewicht von 300 bis 100000 (nach dem Zahlenmittel), in der die Variablen unabhängig voneinander folgende Bedeutung haben:
R¹ Wasserstoff, C₁-C₁₂-Alkyl, R⁶-C(=O)-, R⁶-NH-C(=O)-, Polyalkoholrest;
R⁵ Wasserstoff, C₁-C₁₂-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-, -CH₂-CHOR⁷-CH₂-;
R⁶ C₁-C₁₂-Alkyl;
R⁷ Wasserstoff, C₁-C₁₂-Alkyl, R⁶-C(=O)-, R⁶-NH-C(=O)-;
n 1 bis 8;
s 0;
u 2 bis 2000;
v 0 bis 2000;
w 0 bis 2000;
und
c) gegebenenfalls eines oder mehreren weiteren copolymerisierbaren Monomeren
und anschließender zumindest teilweiser Verseifung der Esterfunktionen der ursprünglichen Monomeren a).

5. Weichkapselhüllen gemäß einem der Ansprüche 1 bis 4, **dadurch gekennzeichnet, daß** die Polymerisate (a) erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I mit einem mittleren Molekulargewicht von 500 bis 50000 (nach dem Zahlenmittel), in der die Variablen unabhängig voneinander folgende Bedeutung haben:
R¹ Wasserstoff, C₁-C₆-Alkyl, R⁶-C(=O)-, R⁶-NH-C(=O)-;
R⁵ Wasserstoff, C₁-C₆-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-, -CH₂-CHOR⁷-CH₂-;
R⁶ C₁-C₆-Alkyl;
R⁷ Wasserstoff, C₁-C₆-Alkyl, R⁶-C(=O)-, R⁶-NH-C(=O)-;
n 1; s 0;
u 5 bis 1000;
v 0 bis 1000;
w 0 bis 1000;
und
c) gegebenenfalls eines oder mehreren weiteren copolymerisierbaren Monomeren
und anschließender zumindest teilweiser Verseifung der Esterfunktionen der ursprünglichen Monomeren a).

6. Weichkapselhüllen gemäß einem der Ansprüche 1 bis 5, **dadurch gekennzeichnet, daß** die Polymerisate (a) erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren weiteren copolymerisierbaren Monomeren
und anschließender zumindest teilweiser Verseifung der Esterfunktionen der ursprünglichen Monomeren a), **dadurch gekennzeichnet, daß** die polyetherhaltigen Verbindungen b) durch Polymerisation von ethylenisch ungesättigten alkylenoxidhaltigen Monomeren und gegebenenfalls weiteren copolymerisierbaren Monomeren hergestellt worden sind.

7. Weichkapselhüllen gemäß Anspruch 6, **dadurch gekennzeichnet, daß** die polyetherhaltigen Verbindungen b) durch Polymerisation von Polyalkylenoxidvinylethern und gegebenenfalls weiteren copolymerisierbaren Monomeren hergestellt worden sind.

8. Weichkapselhüllen gemäß Anspruch 6, **dadurch gekennzeichnet, daß** die polyetherhaltigen Verbindungen b) durch Polymerisation von Polyalkylenoxid(meth)acrylaten und gegebenenfalls weiteren copolymerisierbaren Monomeren hergestellt worden sind.

9. Weichkapselhüllen gemäß einem der Ansprüche 1 bis 8, **dadurch gekennzeichnet, daß** das weitere copolymerisierbare Monomer c) ausgewählt wird aus der Gruppe:
Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie z.B. Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)-propyl]methacrylamid quaternisiert mit Methylchlorid, Methylsulfat oder Diethylsulfat.

10. Weichkapselhüllen gemäß einem der Ansprüche 1 bis 9, **dadurch gekennzeichnet, daß** die Mengenverhältnisse
a) 10 bis 98 Gew.-%
b) 2 bis 90 Gew.-%
c) 0 bis 50 Gew.-%
betragen.

11. Weichkapselhüllen gemäß einem der Ansprüche 1 bis 10, **dadurch gekennzeichnet, daß** die Mengenverhältnisse
a) 50 bis 97 Gew.-%
b) 3 bis 50 Gew.-%
c) 0 bis 20 Gew.-%
betragen.

12. Weichkapselhüllen gemäß einem der Ansprüche 1 bis 11, **dadurch gekennzeichnet, daß** die Mengenverhältnisse
a) 65 bis 97 Gew.-%
b) 3 bis 35 Gew.-%
c) 0 bis 20 Gew.-%
betragen.

13. Weichkapselhüllen gemäß einem der Ansprüche 1 bis 12, **dadurch gekennzeichnet, daß** die erhaltenen Polymerisate nachträglich durch eine polymeranaloge Umsetzung vernetzt werden.

14. Weichkapselhüllen gemäß einem der Ansprüche 1 bis 13, **dadurch gekennzeichnet, daß** für die nachträgliche Vernetzung Dialdehyde, Diketone, Dicarbonsäuren, Borsäure, Borsäuresalze sowie Salze mehrwertiger Kationen eingesetzt werden.

15. Weichkapselhüllen, gemäß einem der Ansprüche 1-14, **dadurch gekennzeichnet, daß** als strukturverbessernde Hilfsstoffe (b) folgende Verbindungsklassen eingesetzt werden:
a)Polymere mit einem Molekulargewicht größer 50000
b) Stoffe, die zu einer Vernetzung der Polymerketten der Polymere führen,
c) sowie gegebenenfalls Stoffen, die zu einer Vernetzung der Polymerketten der strukturverbessernden Hilfsstoffen führen.

16. Weichkapselhüllen gemäß einem der Ansprüche 1-15, **dadurch gekennzeichnet, daß** als strukturverbessernde Hilfsstoffe Polymere aus folgenden Stoffklassen eingesetzt werden:
Polyaminosäuren, wie Gelatine, Zein, Sojaprotein sowie Derivate davon, Polysaccharide wie Stärke, abgebaute Stärke, Maltodextrine, Carboxymethylstärke, Cellulose, Hydroxypropylmethylcellulose, Hydroxypropylcellulose, Hydroxyethylcellulose, Methylcellulose, Carboxymethylcellulose, Ethylcellulose, Celluloseacetat, Celluloseacetatphthalat, Hydroxypropylcelluloseacetatphthalat, Hydroxypropylcelluloseacetatsuccinat, Hemicellulose, Galactomannane, Pectine, Alginate, Carrageenane, Xanthan, Gellan,Dextran,Curdlan, Pullulan, Gummi arabicum, Chitin, sowie Derivate davon, synthetische Polymere wie Polyacrylsäure, Polymethacrylsäure, Copolymerisate aus Acrylsäure- und Methacrylsäureestern, Polyvinylalkohole, Polyvinylacetat, Polyethylenglykole, Polyoxyethylen-Polyoxypropylen-Blockpolymere, Polyvinylpyrrolidone sowie Derivate davon.

17. Weichkapselhüllen gemäß einem der Ansprüche 1 bis 16, **dadurch gekennzeichnet, daß** als weitere übliche Hüllbestandteile Füllstoffe, Formtrennmittel, Rieselhilfsmittel, Farbstoffe, Pigmente, Opakisierungsmittel, Aromen, Süßstoffe, Weichmacher, Konservierungsmittel und/oder Wirkstoffe enthalten sind.

18. Weichkapselhüllen gemäß einem der Ansprüche 1 bis 17, **dadurch gekennzeichnet, daß** die Hülle besteht aus 10 bis 100 % Polymerisaten von Vinylestern auf Polyether gegebenenfalls 0 bis 80 % strukturverbessernden Hilfsstoffen und gegebenenfalls 0 bis 30 % weiteren üblichen Bestandteilen.

19. Weichkapselhüllen gemäß einem der Ansprüche 1 bis 18, erhältlich durch Verfahren wie rotary-die-Verfahren, Accogel-Verfahren, Norton-Verfahren, Tropf- oder Blasverfahren oder das Colton-Upjohn-Verfahren.

20. Weichkapselhüllen gemäß einem der Ansprüche 1 bis 19, **dadurch gekennzeichnet, daß** sie einen oder mehrere pharmazeutische Wirkstoffe, Vitamine, Carotinoide, Mineralstoffe, Spurenelemente, Nahrungsergänzungsstoffe, kosmetische Wirkstoffe, Pflanzenschutzmittel, Badezusätze, Parfüm, Aroma, Reinigungsmittel oder Waschmittel beinhalten.

21. Weichkapselhüllen gemäß einem der Ansprüche 1 bis 20, **dadurch gekennzeichnet, daß** in der Hülle 20 bis 80 %, eines magensaftresistenten Polymers enthalten sind.

22. Weichkapselhüllen gemäß einem der Ansprüche 1 bis 21, **dadurch gekennzeichnet, daß** zur Erzielung einer Magensaftresistenz nach der Herstellung mit pharmazeutisch üblichen Coatingverfahren ein magensaftresistenter Überzug aufgebracht wird.

23. Verwendung der Weichkapselhüllen gemäß einem der Ansprüche 1 bis 22, für pharmazeutische Anwendungen.

24. Verwendung der Weichkapselhüllen gemäß einem der Ansprüche 1 bis 23 für kosmetische Anwendungen, Anwendungen im Pflanzenschutz, für Reinigungsmittel oder Nahrungsergänzungsmittel.

25. Verwendung von Polymerisaten, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren copolymerisierbaren Monomeren
und anschließender zumindest teilweiser Verseifung der Esterfunktionen der ursprünglichen Monomeren a) zur Herstellung von Weichkapselhüllen gemäß einem der Ansprüche 1 bis 23.

26. Verwendung von Polymerisaten, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren weiteren copolymerisierbaren Monomeren
und anschließender zumindest teilweiser Verseifung der Esterfunktionen der ursprünglichen Monomeren a), **dadurch gekennzeichnet, daß** die polyetherhaltigen Verbindungen b) durch Polymerisation von ethylenisch ungesättigten alkylenoxidhaltigen Monomeren und gegebenenfalls weiteren copolymerisierbaren Monomeren hergestellt worden sind, zur Herstellung von Weichkapselhüllen gemäß einem der Ansprüche 1 bis 23.

## Claims

1. A soft capsule shell comprising
(a) polymers prepared by polymerization of vinyl esters in the presence of polyethers
(b) if appropriate structure-improving auxiliaries and
(c) if appropriate other conventional constituents.

2. The soft capsule shell according to claim 1, wherein the polymers (a) are obtainable by free-radical polymerization of
a) at least one vinyl ester of C₁-C₂₄-carboxylic acids in the presence of
b) polyether-containing compounds and
c) if appropriate one or more copolymerizable monomers
and subsequent at least partial hydrolysis of the ester functions in the original monomers a).

3. The soft capsule shell according to either of claims 1 or 2, wherein the polymers (a) are obtainable by free-radical polymerization of
a) at least one vinyl ester of C₁-C₂₄-carboxylic acids in the presence of
b) polyether-containing compounds of the general formula I 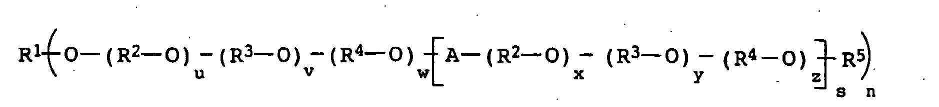 in which the variables have, independently of one another, the following meaning:
R¹ hydrogen, C₁-C₂₄-alkyl, R⁶-C(=O)-, R⁶-NH-C(=O)-, polyalcohol residue;
R⁵ hydrogen, C₁-C₂₄-alkyl, R⁶-C(=O)-, R⁶-NH-C(=O)-;
R² to R⁴ -(CH₂)₂-, -(CH₂)₃-, -(CH₂)₄-, -CH₂-CH(R⁶)-, -CH₂-CHOR⁷-CH₂-;
R⁶ C₁-C₂₄-alkyl;
R⁷ hydrogen, C₁-C₂₄-alkyl, R⁶-C(=O)-, R⁶-NH-C(=O)-;
A -C(=O)-O, -C(=O)-B-C(=O)-O, -C(=O)-NH-B-NH-C(=O)-O;
B -(CH₂)ₜ-, arylene, optionally substituted;
n 1 to 1000;
s 0 to 1000;
t 1 to 12;
u 1 to 5000;
v 0 to 5000;
w 0 to 5000;
x 0 to 5000;
y 0 to 5000;
z 0 to 5000;
and
c) if appropriate one or more other copolymerizable monomers
and subsequent at least partial hydrolysis of the ester functions in the original monomers a).

4. The soft capsule shell according to any of claims 1 to 3, wherein the polymers (a) are obtainable by free-radical polymerization of
a) at least one vinyl ester of C₁-C₂₄-carboxylic acids in the presence of
b) polyether-containing compounds of the general formula I with a number average molecular weight of from 300 to 100000, in which the variables have, independently of one another, the following meaning:
R¹ hydrogen, C₁-C₁₂-alkyl, R⁶-C(=O)-, R⁶-NH-C(=O)-, polyalcohol residue;
R⁵ hydrogen, C₁-C₁₂-alkyl, R⁶-C(=O)-, R⁶-NH-C(=O)-;
R² to R⁴ -(CH₂)₂-, -(CH₂)₃-, -(CH₂)₄-, -CH₂-CH(R⁶)-, -CH₂-CHOR⁷-CH₂-;
R⁶ C₁-C₁₂-alkyl;
R⁷ hydrogen, C₁-C₁₂-alkyl, R⁶-C(=O)-, R⁶-NH-C(=O)-;
n 1 to 8;
s 0;
u 2 to 2000;
v 0 to 2000;
w 0 to 2000;
and
c) if appropriate one or more other copolymerizable monomers
and subsequent at least partial hydrolysis of the ester functions in the original monomers a).

5. The soft capsule shell according to any of claims 1 to 4, wherein the polymers (a) are obtainable by free-radical polymerization of
a) at least one vinyl ester of C₁-C₂₄-carboxylic acids in the presence of
b) polyether-containing compounds of the general formula I with a number average molecular weight of from 500 to 50000, in which the variables have, independently of one another, the following meaning:
R¹ hydrogen, C₁-C₆-alkyl, R⁶-C(=O)-, R⁶-NH-C(=O)-;
R⁵ hydrogen, C₁-C₆-alkyl, R⁶-C(=O)-, R⁶-NH-C(=O)-;
R² to R⁴ -(CH₂)₂-, -(CH₂)₃-, -(CH₂)₄-, -CH₂-CH(R⁶)-, -CH₂-CHOR⁷-CH₂-;
R⁶ C₁-C₆-alkyl;
R⁷ hydrogen, C₁-C₆-alkyl, R⁶-C(=O)-, R⁶-NH-C(=O)-;
n 1;
s 0;
u 5 to 1000;
v 0 to 1000;
w 0 to 1000;
and
c) if appropriate one or more other copolymerizable monomers
and subsequent at least partial hydrolysis of the ester functions in the original monomers a).

6. The soft capsule shell according to any of claims 1 to 5, wherein the polymers (a) are obtainable by free-radical polymerization of
a) at least one vinyl ester of C₁-C₂₄-carboxylic acids in the presence of
b) polyether-containing compounds and
c) if appropriate one or more other copolymerizable monomers
and subsequent at least partial hydrolysis of the ester functions in the original monomers a), wherein the polyether-containing compounds b) have been prepared by polymerization of ethylenically unsaturated alkylene oxide-containing monomers and, if appropriate, other copolymerizable monomers.

7. The soft capsule shell according to claim 6, wherein the polyether-containing compounds b) have been prepared by polymerization of polyalkylene oxide vinyl ethers and, if appropriate, other copolymerizable monomers.

8. The soft capsule shell according to claim 6, wherein the polyether-containing compounds b) have been prepared by polymerization of polyalkylene oxide (meth)acrylates and, if appropriate, other copolymerizable monomers.

9. The soft capsule shell according to any of claims 1 to 8, wherein the other copolymerizable monomer c) is selected from the group of:
acrylic acid, methacrylic acid, maleic acid, fumaric acid, crotonic acid, maleic anhydride and its monoesters, methyl acrylate, methyl methacrylate, ethyl acrylate, ethyl methacrylate, n-butyl acrylate, n-butyl methacrylate, t-butyl acrylate, t-butyl methacrylate, isobutyl acrylate, isobutyl methacrylate, 2-ethylhexyl acrylate, stearyl acrylate, stearyl methacrylate, N-t-butylacrylamide, N-octylacrylamide, 2-hydroxyethyl acrylate, hydroxypropyl acrylates, 2-hydroxyethyl methacrylate, hydroxypropyl methacrylates, alkylene glycol (meth)acrylates, styrene, unsaturated sulfonic acids such as, for example, acrylamidopropanesulfonic acid, vinylpyrrolidone, vinylcaprolactam, vinyl ethers (for example: methyl, ethyl, butyl or dodecyl vinyl ether), vinylformamide, vinylmethylacetamide, vinylamine, 1-vinylimidazole, 1-vinyl-2-methylimidazole, N,N-dimethylaminomethyl methacrylate and N-[3-(dimethylamino)propyl]methacrylamide; 3-methyl-1-vinylimidazolium chloride, 3-methyl-1-vinylimidazolium methyl sulfate, N,N-dimethylaminoethyl methacrylate, N-[3-(dimethylamino)-propyl]methacrylamide quaternized with methyl chloride, methyl sulfate or diethyl sulfate.

10. The soft capsule shell according to any of claims 1 to 9, wherein the ratios of amounts are
a) 10 to 98% by weight
b) 2 to 90% by weight
c) 0 to 50% by weight.

11. The soft capsule shell according to any of claims 1 to 10, wherein the ratios of amounts are
a) 50 to 97% by weight
b) 3 to 50% by weight
c) 0 to 20% by weight.

12. The soft capsule shell according to any of claims 1 to 11, wherein the ratios of amounts are
a) 65 to 97% by weight
b) 3 to 35% by weight
c) 0 to 20% by weight.

13. The soft capsule shell according to any of claims 1 to 12, wherein the resulting polymers are subsequently crosslinked by a polymer-analogous reaction.

14. The soft capsule shell according to any of claims 1 to 13, wherein dialdehydes, diketones, dicarboxylic acids, boric acid, boric acid salts, and salts of multiply charged cations are employed for the subsequent crosslinking.

15. The soft capsule shell according to any of claims 1 to 14, wherein the structure-improving auxiliaries (b) employed are compounds from the following classes:
a) Polymers with a molecular weight of more than 50000
b) substances leading to crosslinking of the polymer chains of the polymers,
c) and, if appropriate, substances which lead to crosslinking of the polymer chains of the structure-improving auxiliaries.

16. The soft capsule shell according to any of claims 1 to 15, wherein the structure-improving auxiliaries employed are polymers from the following classes of substances:
Polyamino acids such as gelatin, zein, soybean protein and derivatives thereof, polysaccharides such as starch, degraded starch, maltodextrins, carboxymethylstarch, cellulose, hydroxypropylmethylcellulose, hydroxypropylcellulose, hydroxyethylcellulose, methylcellulose, carboxymethylcellulose, ethylcellulose, cellulose acetate, cellulose acetate phthalate, hydroxypropylcellulose acetate phthalate, hydroxypropylcellulose acetate succinate, hemicellulose, galactomannans, pectins, alginates, carrageenans, xanthan, gellan, dextran, curdlan, pullulan, gum arabic, chitin, and derivatives thereof, synthetic polymers such as polyacrylic acid, polymethacrylic acid, copolymers of acrylic esters and methacrylic esters, polyvinyl alcohols, polyvinyl acetate, polyethylene glycols, polyoxyethylene/polyoxypropylene block copolymers, polyvinylpyrrolidones and derivatives thereof.

17. The soft capsule shell according to any of claims 1 to 16, wherein other conventional constituents of the shell which are present are fillers, release agents, flow aids, dyes, pigments, opacifiers, flavorings, sweeteners, plasticizers, preservatives and/or active ingredients.

18. The soft capsule shell according to any of claims 1 to 17, wherein the shell consists of from 10 to 100% by weight of polymers of vinyl esters on polyether if appropriate from 0 to 80% of structure-improving auxiliaries and, if appropriate, from 0 to 30% of other conventional constituents.

19. The soft capsule shell according to any of claims 1 to 18, obtainable by processes such as the rotary die process, Accogel process, Norton process, drop or blow process or the Colton-Upjohn process.

20. The soft capsule shell according to any of claims 1 to 19, which comprises one or more active pharmaceutical ingredients, vitamins, carotenoids, minerals, trace elements, food supplements, cosmetic active ingredients, crop protection agents, bath additives, perfume, flavoring, cleaner or detergent.

21. The soft capsule shell according to any of claims 1 to 20, wherein the shell comprises from 20 to 80% of a polymer resistant to gastric fluid.

22. The soft capsule shell according to any of claims 1 to 21, wherein resistance to gastric fluid is achieved by applying after production a coating resistant to gastric fluid by conventional pharmaceutical coating processes.

23. The use of the soft capsule shells according to any of claims 1 to 22 for pharmaceutical applications.

24. The use of the soft capsule shells according to any of claims 1 to 23 for cosmetic applications, applications in crop protection, for cleaners or food supplements.

25. The use of polymers obtainable by free-radical polymerization of
a) at least one vinyl ester of C₁-C₂₄-carboxylic acids in the presence of
b) polyether-containing compounds and
c) if appropriate one or more copolymerizable monomers
and subsequent at least partial hydrolysis of the ester functions in the original monomers a) to produce soft capsule shells according to any of claims 1 to 23.

26. The use of polymers obtainable by free-radical polymerization of
a) at least one vinyl ester of C₁-C₂₄-carboxylic acids in the presence of
b) polyether-containing compounds and
c) if appropriate one or more copolymerizable monomers
and subsequent at least partial hydrolysis of the ester functions in the original monomers a), wherein the polyether-containing compounds b) have been prepared by polymerization of ethylenically unsaturated alkylene oxide-containing monomers and, if appropriate, other copolymerizable monomers, for producing soft capsule shells according to any of claims 1 to 23.

## Revendications

1. Enveloppes de capsules souples contenant :
(a) des polymères fabriqués par polymérisation d'esters de vinyle en présence de polyéthers,
(b) éventuellement des agents auxiliaires renforçant la structure et
(c) éventuellement d'autres composants usuels.

2. Enveloppes de capsules souples selon la revendication 1, **caractérisées en ce que** les polymères (a) peuvent être obtenus par polymérisation radicalaire :
a) d'au moins un ester vinylique d'acides carboxyliques en C₁-C₂₄ en présence
b) de composés à teneur en polyéthers et
c) éventuellement d'un ou plusieurs monomères copolymérisables,
suivie d'une saponification au moins partielle des fonctions ester des monomères d'origine a).

3. Enveloppes de capsules souples selon l'une quelconque des revendications 1 ou 2, **caractérisées en ce que** les polymères (a) peuvent être obtenus par polymérisation radicalaire
a) d'au moins un ester vinylique d'acides carboxyliques en C₁-C₂₄ en présence
b) de composés à teneur en polyéthers de formule générale I :
R¹ (̵O- (R²-O)ᵤ -(R³-O)ᵥ -(R⁴-O)_{w} ⁅A- (R²-O)ₓ - (R³-O)_{y} - (R⁴-O)_{z} ⁆ₛ R⁵)ₙ
dans laquelle les variables ont, indépendamment l'une de l'autre, la signification suivante :
R¹ est l'hydrogène, un alkyle en C₁-C₂₄, R⁶-C(=O)-, R⁶-NH-C(=O)-, un radical polyalcool;
R⁵ est l'hydrogène, un alkyle en C₁-C₂₄, R⁶-C(=O)-, R⁶-NH-C (=O) -;
R² à R⁴ désignent
-(CH₂)₂-, -(CH₂)₃-, -(CH₂)₄-, -CH₂-CH(R⁶)-, -CH₂₋CHOR⁷-CH₂-;
R⁶ est un alkyle en C₁-C₂₄;
R⁷ est l'hydrogène, un alkyle en C₁-C₂₄, R⁶-C(=O)-, R6-NH-C(=O)-;
A est -C(=O)-O, -C(=O)-B-C(=O)-O, -C(=O)-NH-B-NH-C(=O)-O;
B est -(CH₂)ₜ-, un arylène éventuellement substitué;
n est 1 à 1000;
s est 0 à 1000;
t est 1 à 12;
u est 1 à 5000;
v est 0 à 5000;
w est 0 à 5000;
x est 0 à 5000;
y est 0 à 5000;
z est 0 à 5000;
et
c) éventuellement d'un ou plusieurs autres monomères copolymérisables,
le tout étant suivi d'une saponification au moins partielle des fonctions ester des monomères d'origine a).

4. Enveloppes de capsules souples selon l'une quelconque des revendications 1 à 3, **caractérisées en ce que** les polymères (a) peuvent être obtenus par polymérisation radicalaire
a) d'au moins un ester vinylique d'acides carboxyliques en C₁-C₂₄ en présence
b) de composés à teneur en polyéthers de formule générale I d'un poids moléculaire moyen de 300 à 100 000 (moyenne en nombre), dans laquelle les variables ont, indépendamment l'une de l'autre, la signification suivante :
R¹ est l'hydrogène, un alkyle en C₁-C₁₂, R⁶-C(=O)-, R⁶-NH-C(=O)-, un radical polyalcool;
R⁵ est l'hydrogène, un alkyle en C₁-C₁₂, R⁶-C(=O)-, R⁶-NH-C (=O)- ;
R² à R⁴ désignent
-(CH₂)₂-, -(CH₂)₃-, -(CH₂)₄-, -CH₂-CH(R⁶)-, -CH₂₋CHOR⁷-CH₂- ;
R⁶ est un alkyle en C₁-C₁₂;
R⁵ est l'hydrogène, un alkyle en C₁-C₁₂, R⁶-C(=O)-, R⁶-NH-C (=O) -;
n est 1 à 8;
s est 0;
u est 2 à 2000;
v est 0 à 2000;
w est 0 à 2000;
et
c) éventuellement d'un ou plusieurs autres monomères copolymérisables,
suivie d'une saponification au moins partielle des fonctions ester des monomères d'origine a).

5. Enveloppes de capsules souples selon l'une quelconque des revendications 1 à 4, **caractérisées en ce que** les polymères (a) peuvent être obtenus par polymérisation radicalaire
a) d'au moins un ester vinylique d'acides carboxyliques en C₁-C₂₄ en présence
b) de composés à teneur en polyéthers de formule générale I d'un poids moléculaire moyen de 500 à 50 000 (moyenne en nombre), dans laquelle les variables ont, indépendamment l'une de l'autre, la signification suivante :
R¹ est l'hydrogène, un alkyle en C₁-C₆ , R⁶-C(=O)-, R⁶-NH-C(=O)- ;
R⁵ est l'hydrogène, un alkyle en C₁-C₆, R⁶-C(=O)-, R⁶-NH-C(=O)-;
R² à R⁴ désignent
-(CH₂)₂-, -(CH₂)₃-, -(CH₂)₄-, -CH₂-CH(R⁶)-, -CH₂₋CHOR⁷-CH₂-;
R⁶ est un alkyle en C₁-C₆;
R⁷ est l'hydrogène, alkyle en C₁-C₆, R⁶-C(=O)-, R⁶-NH-C(=O)-;
n est 1;
s est 0;
u est 5 à 1000;
v est 0 à 1000;
w est 0 à 1000;
et
c) éventuellement d'un ou plusieurs autres monomères copolymérisables,
le tout étant suivi d'une saponification au moins partielle des fonctions ester des monomères d'origine a).

6. Enveloppes de capsules souples selon l'une quelconque des revendications 1 à 5, **caractérisées en ce que** les polymères (a) peuvent être obtenus par polymérisation radicalaire
a) d'au moins un ester vinylique d'acides carboxyliques en C₁-C₂₄ en présence
b) de composés à teneur en polyéthers et
c) éventuellement d'un ou plusieurs autres monomères copolymérisables,
suivie d'une saponification au moins partielle des fonctions ester des monomères d'origine a), **caractérisées en ce que** les composés à teneur en polyéthers b) ont été fabriqués par polymérisation de monomères à teneur en oxydes d'alkylène éthyléniquement insaturés et éventuellement d'autres monomères copolymérisables.

7. Enveloppes de capsules souples selon la revendication 6, **caractérisées en ce que** les composés à teneur en polyéthers b) ont été fabriqués par polymérisation d'éthers vinyliques de poly(oxydes d'alkylène) et éventuellement d'autres monomères copolymérisables.

8. Enveloppes de capsules souples selon la revendication 6, **caractérisées en ce que** les composés à teneur en polyéthers b) ont été fabriqués par polymérisation de (méth)acrylates de poly(oxydes d'alkylène) et éventuellement d'autres monomères copolymérisables.

9. Enveloppes de capsules souples selon l'une quelconque des revendications 1 à 8, **caractérisées en ce que** l'autre monomère copolymérisable c) est sélectionné dans le groupe suivant :
l'acide acrylique, l'acide méthacrylique, l'acide maléique, l'acide fumarique, l'acide crotonique, l'anhydride d'acide maléique ainsi que ses semi-esters, l'acrylate de méthyle, le méthacrylate de méthyle, l'acrylate d'éthyle, le méthacrylate d'éthyle, l'acrylate de n-butyle, le méthacrylate de n-butyle, l'acrylate de t-butyle, le méthacrylate de t-butyle, l'acrylate d'isobutyle, le méthacrylate d'isobutyle, l'acrylate de 2-éthylhexyle, l'acrylate de stéaryle, le méthacrylate de stéaryle, l'acrylamide de N-t-butyle, l'acrylamide de N-octyle, l'acrylate de 2-hydroxyéthyle, l'acrylate d'hydroxy-propyle, le méthacrylate de 2-hydroxyéthyle, le méthacrylate d'hydroxypropyle, le (méth)acrylate d'alkylèneglycol, le styrène, les acides sulfoniques insaturés tels que l'acide acrylamidopropanesulfonique, la vinylpyrrolidone, le vinylcaprolactame, les éthers vinyliques (par exemple, l'éther méthyl-, éthyl-, butyl- ou dodécylvinylique) et le vinylformamide, le vinylméthylacétamide, la vinylamine, le 1-vinylimidazole, le 1-vinyl-2-méthylimidazole, le méthacrylate de N,N-diméthylaminométhyle et le méthacrylamide de N-[3-(dimethylamino)propyle]; le chlorure de 3-méthyl-1-vinylimidazolium, le méthylsulfate de 3-méthyl-1-vinyl-imidazolium, le méthacrylate de N,N-diméthyl-aminoéthyle, le méthacrylamide de N-[3-(diméthylamino)-propyle] quaternisé avec du chlorure de méthyle, du sulfate de méthyle ou du sulfate de diéthyle.

10. Enveloppes de capsules souples selon l'une quelconque des revendications 1 à 9, **caractérisées en ce que** les rapports quantitatifs sont les suivants :
a) 10 à 98% en poids
b) 2 à 90% en poids
c) 0 à 50% en poids.

11. Enveloppes de capsules souples selon l'une quelconque des revendications 1 à 10, **caractérisées en ce que** les rapports quantitatifs sont les suivants :
a) 50 à 97% en poids
b) 3 à 50% en poids
c) 0 à 20% en poids.

12. Enveloppes de capsules souples selon l'une quelconque des revendications 1 à 11, **caractérisées en ce que** les rapports quantitatifs sont les suivants :
a) 65 à 97% en poids
b) 3 à 35% en poids
c) 0 à 20% en poids.

13. Enveloppes de capsules souples selon l'une quelconque des revendications 1 à 12, **caractérisées en ce que** les polymères obtenus sont ensuite réticulés par une réaction de type polymérisation.

14. Enveloppes de capsules souples selon l'une quelconque des revendications 1 à 13, **caractérisées en ce que**, pour la réticulation ultérieure, on utilise des dialdéhydes, des dicétones, des acides dicarboxyliques, l'acide borique, des sels d'acide borique et des sels de cations polyvalents.

15. Enveloppes de capsules souples selon l'une quelconque des revendications 1 à 14, **caractérisées en ce que** l'on utilise les classes de composés suivantes comme agents auxiliaires (b) renforçant la structure :
a) des polymères d'un poids moléculaire supérieur à 50 000,
b) des substances menant à une réticulation des chaînes de polymères,
c) ainsi qu'éventuellement des substances menant à une réticulation des chaînes polymères des agents auxiliaires renforçant la structure.

16. Enveloppes de capsules souples selon l'une quelconque des revendications 1 à 15, **caractérisées en ce que** l'on utilise des polymères des classes de substances suivantes comme agents auxiliaires renforçant la structure :
des acides polyaminés, tels que la gélatine, la zéine, la protéine de soja ainsi que leurs dérivés, des polysaccharides, tels que l'amidon, l'amidon dégradé, la maltodextrine, l'amidon de carboxyméthyle, la cellulose, l'hydroxypropylméthylcellulose, l'hydroxypropylcellulose, l'hydroxyéthylcellulose, la méthylcellulose, la carboxyméthylcellulose, l'éthylcellulose, l'acétate de cellulose, le phtalate acétate de cellulose, le phtalate acétate d'hydroxypropylcellulose, le succinate acétate d'hydroxypropylcellulose, l'hémicellulose, le galactomannane, la pectine, les alginates, le carraghénane, le xanthane, le gellane, le dextrane, le curdlane, le pullulane, la gomme arabique, la chitine, ainsi que leurs dérivés, des polymères de synthèse, tels que l'acide polyacrylique, l'acide polyméthacrylique, des copolymères d'esters d'acide acrylique et d'acide méthacrylique, des alcools polyvinyliques, le poly(acétate de vinyle), les polyéthylèneglycols, les polymères séquencés de polyoxyéthylène et de polyoxypropylène, les polyvinylpyrrolidones ainsi que leurs dérivés.

17. Enveloppes de capsules souples selon l'une quelconque des revendications 1 à 16, **caractérisées en ce qu'**elles contiennent, comme autres composants habituels des enveloppes, des charges, des agents de démoulage, des agents auxiliaires de ruissellement, des colorants, des pigments, des agents opacifiants, des arômes, des édulcorants, des agents plastifiants, des conservateurs et/ou des principes actifs.

18. Enveloppes de capsules souples selon l'une quelconque des revendications 1 à 17, **caractérisées en ce que** l'enveloppe est constituée de 10 à 100 % de polymères de vinylesters sur des polyéthers, éventuellement de 0 à 80% d'agents auxiliaires renforçant la structure et éventuellement de 0 à 30% d'autres composants usuels.

19. Enveloppes de capsules souples selon l'une quelconque des revendications 1 à 18, pouvant être obtenues par des procédés tels que les procédés rotary-die, Accogel, Norton, un procédé d'égouttement ou de soufflage ou Colton-Upjohn.

20. Enveloppes de capsules souples selon l'une quelconque des revendications 1 à 19, **caractérisées en ce qu'**elles contiennent un ou plusieurs principes actifs pharmaceutiques, des vitamines, des caroténoïdes, des substances minérales, des oligoéléments, des compléments alimentaires, des principes actifs cosmétiques, des agents phytosanitaires, des additifs de bain, du parfum, des arômes, des produits de nettoyage ou de lavage.

21. Enveloppes de capsules souples selon l'une quelconque des revendications 1 à 20, **caractérisées en ce que** l'enveloppe contient 20 à 80% d'un polymère résistant au suc gastrique.

22. Enveloppes de capsules souples selon l'une quelconque des revendications 1 à 21, **caractérisées en ce que**, pour obtenir une résistance au suc gastrique, on applique après fabrication un enrobage résistant au suc gastrique avec un procédé d'enrobage habituel en pharmacie.

23. Utilisation des enveloppes de capsules souples selon l'une quelconque des revendications 1 à 22 pour applications pharmaceutiques.

24. Utilisation des enveloppes de capsules souples selon l'une quelconque des revendications 1 à 23 pour applications cosmétiques, applications phytosanitaires, produits de nettoyage ou compléments alimentaires.

25. Utilisation de polymères qui peuvent être obtenus par polymérisation radicalaire
a) d'au moins un ester vinylique d'acides carboxyliques en C₁-C₂₄ en présence
b) de composés à teneur en polyéthers et
c) éventuellement d'un ou plusieurs monomères copolymérisables,
suivie d'une saponification au moins partielle des fonctions ester des monomères d'origine a) pour la fabrication d'enveloppes de capsules souples selon l'une quelconque des revendications 1 à 23.

26. Utilisation de polymères qui peuvent être obtenus par polymérisation radicalaire
a) d'au moins un ester vinylique d'acides carboxyliques en C₁-C₂₄ en présence
b) de composés à teneur en polyéthers et
c) éventuellement d'un ou plusieurs autres monomères copolymérisables,
suivie d'une saponification au moins partielle des fonctions ester des monomères d'origine a), **caractérisée en ce que** les composés b) à teneur en polyéthers ont été fabriqués par polymérisation de monomères à teneur en oxydes d'alkylène éthyléniquement insaturés et éventuellement d'autres monomères copolymérisables pour la fabrication d'enveloppes de capsules souples selon l'une quelconque des revendications 1 à 23.
